# EUROPÄISCHE PATENTSCHRIFT

(11) **EP 1 084 268 B1**
(45) Veröffentlichungstag und Bekanntmachung des Hinweises auf die Patenterteilung: **02.04.2003**
(21) Anmeldenummer: 99926342.9
(22) Anmeldetag: 20.05.1999
(51) Int. Cl.: C12P 21/02, C12N 1/20

(54) **BIOTECHNOLOGISCHE HERSTELLUNG VON POLYGLUTAMINSÄURE**
BIOTECHNOLOGICAL PRODUCTION OF POLYGLUTAMINIC ACID
PRODUCTION D'ACIDE POLYGLUTAMINIQUE PAR DES METHODES BIOTECHNOLOGIQUES

(30) Priorität: 02.06.1998 DE 19825507
(43) Veröffentlichungstag der Anmeldung: 21.03.2001
(73) Patentinhaber: Bayer Aktiengesellschaft, 51368 Leverkusen (DE)
(72) Erfinder: STEINBÜCHEL, Alexander, D-48341 Altenberge (DE); REHM, Bernd, Helmut, Adam, D-48301 Nottuln-Schapdetten (DE); HEZAYEN, Francis Fakhry, Horstmarer Landweg 84, 48149 Münster (DE); OPPERMANN-SANIO, Fred, Bernd, D-59075 Hamm (DE); JOENTGEN, Winfried, D-51067 Köln (DE)
(86) Internationale Anmeldenummer: EP9903461
(87) Internationale Veröffentlichungsnummer: WO99063106

(56) Entgegenhaltungen:
- EP-A- 0 561 452
- EP-A- 0 561 464
- TATSUO WATANABE ET AL: "Effective formation of Di- and Tri-gamma-glutamates by gamma-glutamyltranspeptidase" BIOSCIENCE BIOTECHNOLOGY BIOCHEMISTRY., Bd. 59, Nr. 1, 1995, Seiten 115-116, XP002120306 JAPAN SOC. FOR BIOSCIENCE, BIOTECHNOLOGY AND AGROCHEM. TOKYO, JP ISSN: 0916-8451
- S.A. GIANNOS ET AL: "The biosynthesis of unusual polyamides containing glutamic acid" PROCEEDINGS OF THE ASC DIVISION OF POLYMERIC MATERIALS SCIENCE AND ENGINEERING, Bd. 62, 1990, Seiten 236-240, XP002103913
- A.-M. CROMWICK ET AL: "Effects of pH and aeration on gamma-polyglutamic acid) formation by Bacillus licheniformis in controlled Batch Fermentor cultures" BIOTECHNOLOGY AND BIOENGINEERING. INCLUDING: SYMPOSIUM BIOTECHNOLOGY IN ENERGY PRODUCTION AND CONSERVATION., Bd. 50, 1996, Seiten 222-227, XP002103912 JOHN WILEY & SONS. NEW YORK., US ISSN: 0006-3592

## Beschreibung

Die Erfindung bezieht sich auf ein Verfahren zur Herstellung von Polyglutaminsäure und ihrer Salze durch einen biotechnologischen Prozeß unter Verwendung von Archaebakterien.

Kristallisations- und Agglomerationsvorgänge gehören als biologische Mineralisation zu den grundlegenden Prozessen in der belebten Natur. So sind sie beispielsweise am Skelett- oder Schalenaufbau in lebenden Organismen beteiligt. In der Natur werden diese Mineralisationsvorgänge durch natürlich vorkommende Proteine und Polysaccharide reguliert. (S. Weiner, Biochem. 22, (1983), 4139 -45; C. S. Sikes, A. P. Wheeler, in Chemical Aspects of Regulation of Mineralisation., Eds. C. S. Sikes , A. P. Wheeler University of South Alabama Publ. Services (1988), 15 - 20).

Unglücklicherweise treten sowohl in der Natur als auch im industriellen Bereich nicht erwünschte Mineralisationsvorgänge auf und führen zu hartnäckigen störenden Niederschlägen und Verkrustungen, z.B. Verkrustungen an Wärmetauscheroberflächen oder Kühltürmen Partikelagglomerationen in Pigmentdispersionen, Verkrustungen auf harten (z.B. Glas Metall) und weichen (Textil) Oberflächen.

In der Vergangenheit wurden verschiedene Vorschläge zur Verhinderung oder Beseitigung dieser Ablagerungen gemacht. So beschreiben die Patente US 4 534 881, US 4 585 560, US 4 587 021 die Inhibierung von Calciumcarbonat-Niederschlägen durch Proteinfraktionen, Polysaccharidfraktionen oder Polyaminosäurefraktionen aus Calciumcarbonat bildenden Organismen wie Crustaceen etc.

Weiterhin wird in der Literatur die Inhibierung mineralischer Niederschläge durch polyanionische hydrophobe Polypeptide mit Block-Copolymer Struktur und verwandte phosphorylierte Polypeptide (US 4 868 287) geschildert. Die verwendeten Polypeptide werden durch peptidchemische Methoden hergestellt (WO 92/17194).

Mögliche Anwendungsgebiete der wasserlöslichen polyanionischen Polymere sind u.a. die Beeinflussung des Kristallisations- oder Agglomerationsverhaltens von schwerlöslichen Salzen oder Feststoffen in wäßrigen Systemen, wie beispielsweise in den folgenden Literaturzitaten beschrieben: S. Sarig et al., seawater Desalin., 150-7, (1977); S. Sarig et al., Invest. Urol., 18, 149 -150 (1980). In der Technik ist der Einsatz z. B. in Geschirrspülmitteln beschrieben EP 0 561 452 und EP 0 561 464 (Unilever).

Bislang sind aus der Natur drei unterschiedliche Polyaminosäuren (Poly-γ-Glutamat, Poly-ε-Lysin und Poly-α-Arginylaspartat (Cyanophycin)) bekannt. Poly-γ-Glutamat wird von verschiedenen Gram-positiven Bakterien wie z.B. Bacillus licheniformis, Bacillus subtilis natto, oder Bacillus anthracis produziert. Poly-ε-Lysin wird von Streptomyces albulus hergestellt. Poly-α-Arginylaspartat wird von vielen Cyanobakterien wie beispielsweise Spirulina platensis, Aphanocapsa PCC 6308 oder Anabena cylindrica produziert.

Die Synthese von Polyglutaminsäure erfolgt auf einem nicht-ribosomalem Weg, wobei ein polyamidisches Polycarboxylat mit polydisperser Molekulargewichtsverteilung erhalten und als Schleim auf der Zelloberfläche abgelegt wird. Die Verknüpfung der Glutaminsäuremonomere wird hierbei zwischen der Aminogruppe und γ-Carboxyl-gruppe durchgeführt (Poly-γ-Glutamat).

In den Arbeiten von M. Bovarnick et al. J. Biol. Chem., 145, 415 (1942) finden sich die frühesten Verfahren zur Herstellung von Polyglutaminsäure unter Einsatz von Mikroorganismen. Ferner ist aus JP 1-174397 (1989), JP 43-24472 (1969), Giannos, S.A., et al. *(Proceedings of the ASC Division of Polymeric Materials Science and Engineering,* (1990), 62, pp.236-240), Cromwick, A.-M., et al. *(Biotechnology and Bioengineering*, (1996), 50, pp.222-227(John Wiley & Sons. NY, US)), und US 2 895 882 die biotechnische Herstellung von Poly-γ-Glulamat unter Verwendung von Bacillus subtilis bzw. Bacillus licheniformis bekannt. Allen Verfahren gemeinsam ist, daß sie mit Eubakterien arbeiten und daher besondere Kulturbedingungen eingehalten werden müssen. Insbesondere muß nach dem bisher bekannten Stand der Technik unter sterilen Bedingungen gearbeitet werden.

Aufgabe der vorliegenden Erfindung ist es daher, ein Verfahren zur Herstellung von Polyglutaminsäure zur Verfügung zu stellen, das die geschilderten Nachteile des Standes der Technik nicht aufweist. Insbesondere soll die biotechnische Herstellung unter möglichst einfachen verfahrenstechnischen Bedingungen erfolgen. Der Meß-, Steuer- und Regelaufwand soll möglichst gering gehalten werden. Vor allem sollen die Anforderungen an die Einhaltung steriler Bedingungen so niedrig sein, daß das Verfahren auch in einem offenen System durchführbar ist.

Diese Aufgabe wird erfindungsgemäß überraschend dadurch gelöst, daß Archaebakterien enthaltende Kulturen eingesetzt werden.

Die erfindungsgemäß einsetzbaren Archaebakterien sind durch eine Reihe allgemeiner Merkmale charakterisiert:
1. Die Zellwand enthält kein Murein, sondern Pseudomurein oder Proteine und Polysaccharide. Die Archaebakterien sind dadurch unempfindlich gegen β-Lactam-Antibiotika.
2. In der Cytoplasmamembran sind keine Fettsäure-Glycerinester vorhanden, sondern Glycerinether mit C₂₀ (Phytanyl)- und C₄₀ (Biphytanyl) isoprenoidalkylen.
3. Die tRNA enthält Ribothymidin Pseudouridin oder 1- Methylpseudouridin.

Durch die genannten Merkmale unterscheiden sich die Archaebakterien von den anderen Prokaryonten (Eubakterien) und Eukaryonten. Hinzu kommt, daß sich die Sequenzen der 5S, 16S und 23S rRNA von denjenigen der Eubakterien und Eukaryonten unterscheiden. Außerdem zeichnen sich die Archaebakterien durch die folgenden weiteren Merkmale aus:
- Der Elongationsfaktor (EF2) enthält Aminosäure-Diphthamid und ist so durch Diphterie-Toxin ADP- ribosylierbar.
- die Aminosäure-Sequenz des ribosomalen A-Proteins weist Sequenzhomologien mit dem entsprechenden eukaryotischen (L-7/L-12) Protein auf.
- die Methionin - Initiator-tRNA wird formyliert.
- einige tRNA-Gene enthalten Introns.

Diese Merkmale haben die Archaebakterien mit den Eukaryonten gemeinsam. Andererseits unterscheiden sich beide hierdurch von den Eubakterien.

Dic Archaebakterien besiedeln im allgemeinen extreme Standorte. Dort leben sie terrestrisch oder aquatisch in anaerober, hypersaliner hydrothermal oder geothermal aufgeheizter Umgebung. Daneben gibt es auch Arten, die aerob oder fakultativ anaerob leben können. Ebenso gibt es Archaebakterien, die chemolitho-autotrophe, organotrophe oder fakultativ organotrophe Lebensweise zeigen.

Die Archaebakterien können sowohl mesophil als auch thermophil sein. Demgemäß können die erfindungsgemäß eingesetzten Archacbakterien bei Temperaturen von 25 bis 50°C, vorzugsweise bei 30 bis 45°C kultiviert werden. Je nach eingesetzter Art können die Archaebakterienkulturen unter aeroben oder anaeroben Bedingungen eingesetzt werden.

Grundsätzlich lassen sich zwei Zweige an Archaebakterien unterscheiden:
- Methanogener Zweig: Methanogene, extrem halophile Archaebakterien (Thermoplasmales, Thermacoccales, Archaeglobales).
- Zweig der thermophilen, Schwefel-methabolisierenden Archaebakterien (Sulfolobales, Thermoproteales, Pyrodictium).

Vorzugsweise werden erfindungsgemäß halophile Archaebakterien eingesetzt. Dabei hat sich als besonders effizient ein Archaebakterium erwiesen, daß unter der Nr. DSM 11921 bei der DSMZ, Deutsche Sammlung von Mikroorganismen und Zellkulturen GmbH in Braunschweig hinterlegt worden ist.

Dieser Organismus ist dadurch gekennzeichnet, daß er in einem Nährmedium wächst, welches große Mengen Natriumchlorid enthält. Vorzugsweise liegen im Nährmedium mehr als 8 % (w/v) Natriumchlorid vor. Außerdem ist das Archaebakterium gegen Antibiotika (z.B. Penicillin oder Chloramphenicol) unempfindlich. Die betreffenden Antibiotika sind andererseits gegen Eubakterien wirksam.

Die erfindungsgemäß einsetzbaren Archaebakterien können in Form von Reinkulturen oder im Gemisch mit anderen Mikroorganismen verwendet werden. Vorzugsweise kommen hierbei verschiedene Arten von Archaebakterien zum Einsatz. Ebenso ist aber auch die Verwendung einer oder mehrerer Arten von Archaebakterien in Kombination mit weiteren Mikroorganismenarten, d.h. Eukaryonten oder Prokaryonten möglich, sofern diese unter den für Archaebakterien bevorzugten Bedingungen kultivierbar sind. So müssen im Falle des erfindungsgcmäß besonders bevorzugten Einsatzes von halophilen Archaebakterien die etwaig zugesetzten weiteren Mikroorganismen in der Lage sein, auch die im Nährmedium vorhandenen hohen Salzgehalte zu tolerieren. Bevorzugt ist erfindungsgemäß jedoch der Einsatz von Reinkulturen einer Art von Archaebakterien. Besonders bevorzugt ist hierbei der Einsatz des genannten Organismus, der unter der Nr. DSM 11921 hinterlegt worden ist.

Durch ihre besonderen Eigenschaften erlauben die erfindungsgemäß einsetzbaren Archaebakterien eine einfache Anzucht und Kultivierung unter nicht sterilen Bedingungen, wodurch der verfahrenstechnische Aufwand bei der Produktion verringert wird. Insbesondere ist es erfindungsgemäß überraschend möglich, eine Produktion von Polyglutaminsäure in einem offenen System durchzuführen.

Vorteilhaft ist weiterhin, daß sich preisgünstige Nährmedien einsetzen lassen. Demgemäß lassen sich erfindungsgemäß alle üblichen, für Archaebakterien geeignete Nährmedien einsetzen. Die Zusammensetzung der Nährmedien variiert auch in Abhängigkeit davon, ob Reinkulturen oder Mischkulturen eingesetzt werden.

Beispielsweise können erfindungsgemäß synthetische, halbsynthetische oder komplexe Kulturmedien in Betracht kommen. Diese können kohlenstoffhaltige und stickstoffhaltige Verbindungen, anorganische Salze, gegebenenfalls Spurenelemente sowie Vitamine enthalten.

Als kohlenstoffhaltige Verbindungen können Kohlenhydrate, Kohlenwasserstoffe, organischc Grundchemikalien oder komplexe Gemische in Betracht kommen. Zu den komplexen Gemischen zählen z.B. Malzextrakt, Hefeextrakt, Casein oder Caseinhydrolysat. Die erfindungsgemäß bevorzugten Nährmedien enthalten vorzugsweise Hefeextrakt, Proteose Pepton oder Casaminsäuren oder Gemische von wenigstens zwei der genannten Bestandteile. Sofern die Kultivierung in einem flüssigen Nährmedium erfolgt, werden vorzugsweise Casaminsäuren eingesetzt. Diese sollen nach einer bevorzugten Ausführungsform Proteose Pepton ersetzen.

Als stickstoffhaltige Nährmcdien können anorganische Verbindungen in Betracht kommen. Beispiele hierfür sind Nitrate und Ammoniumsalze. Ebenso können organische Stickstoffquellen zum Einsatz kommen. Hierzu zählen zum Beispiel Hefeextrakt, Sojamehl, Casein und Caseinhydrolysat. Möglich ist auch der Einsatz zweier oder mehrerer der genannten Verbindungen als Gemisch.

Zu den einsetzbaren anorganischen Salze zählen beispielsweise Sulfate, Nitrate, Chloride, Carbonate und Phoshate. Als Metalle enthalten die genannten Salze vorzugsweise Natrium, Kalium, Magnesium, Mangan, Calcium, Zink und Eisen. Erfindungsgemäß kann auch ein Gemisch zweier oder mehrerer der genannten Salze verwendet werden. Erfindungsgemäß bevorzugt wird ein Nährmedium mit einem hohen Anteil an anorganischen Salzen.

Die erfindungsgemäß einsetzbaren Archaebakterienkulturen lassen sich in den üblichen Fermentationsvorrichtungen zur Gewinnung der Polyglutaminsäure kultivieren. In Betracht kommen demgemäß sowohl für die Oberflächenkultivierung als auch für Submerskulturen geeignete Bioreaktoren. Ebenso sind Festbettreaktoren und Reaktoren mit homogen gemischten Nährmedien geeignet. Beispiele für einsetzbare Reaktoren sind u. a. Rührreaktoren, Blasensäulen, Schlaufenreaktoren und ähnliche Systeme.

Vorteilhafterweise können die genannten Reaktoren als offene Systeme ausgestaltet sein; denn die Kultivierung erfordert nicht sterile Bedingungen. Insbesondere bei Einsatz von Nährmedien mit dem o.g. hohen Anteil an Natriumchlorid ist trotz der Gegenwart von komplexen Bestandteilen das alleinige Wachstum der Archaebakterien gewährleistet. Im Unterschied zu allen bisher bekannten biotechnologischen Verfahren zur Polyglutaminsäuregewinnung kann daher die Zeit, Energie- und kostenintensive Sterilisation der Medien und die kostenintensive sterile Prozeßführung bei der Kultivierung unterbleiben.

Zur Herstellung von Polyglutaminsäure werden die Archaebakterien enthaltenden Kulturen ca. 1 bis 4, vorzugsweise ca. 2 Tage kultiviert. Danach wird das Produkt aus dem Nährmedium in üblicher Weise isoliert. Vorzugsweise handelt es sich hierbei um Polymere der folgenden Struktur:

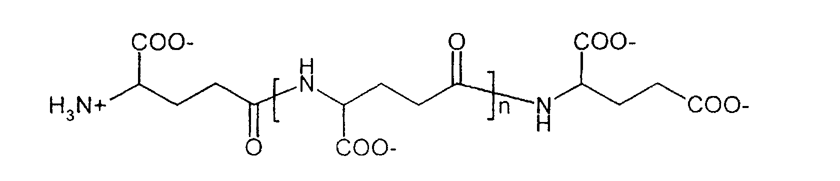

Das Molekulargewicht der Polymere beträgt vorzugsweise > 100 000.

Das Polymer kann sowohl von bewachsenem Festmedium als auch aus bewachsener Flüssigkultur gewonnen werden. Im zuerst genannten Fall wird der von den Organismen gebildete Schleim von der Oberfläche des festen Nährmedien abgeschabt. In einer bevorzugten Ausführungsform wird sodann eine Suspension, vorzugsweise in Alkohol, des Schleims hergestellt. Aus dieser Suspension kann anschließend das Polymer ausgefällt werden.

In gleicher Weise wird bei Durchführung der Kultivierung im Submersverfahren die Produktgewinnung durchgeführt. Hier kann nämlich nach der Abtrennung der Zellen aus dem flüssigen Nährmedium ebenfalls durch Zugabe von Alkohol und anschließendes Ausfällen das Polymer gewonnen werden.

im folgenden wird die Erfindung unter Bezugnahme auf die Beispiele näher erläutert:

### Beispiel 1

### Isolierung des Polymers von Festmedium

Kultivierungsbedingungen: Das Bakterium wurde auf Festmedium (Tabelle) ausgestrichen und bei 42°C für vier Tage inkubiert. Die Kultivierung erfolgte unter unsterilen Bedingungen. Wiederholte Kontrollausstriche zum Ende der Kultivierungszeit auf Standard I-Festmedium (Fa. Merck, Darmstadt) haben keinen Hinweis auf Kontaminationen erbracht. Unter den aufgeführten Bedingungen gewährleistet die hohe Salzkonzentration trotz der Gegenwart großer Mengen an komplexen Nährmedienbestandteilen das alleinige Wachstum des Archaebakteriums.

**Tabelle**

| Zusammensetzung des Mediums 1. Der pH-Wert wurde mit 4 N NaOH auf 7.2 eingestellt. Das Medium wurde durch Erhitzen in einem herkömmlichen Mikrowellenherd in Lösung gebracht und direkt in Petrischalen gefüllt. | |
|---|---|
| **Komponente** | **Zusatz pro Liter Endvolumen [g]** |
| NaCl | 225 |
| MgSO₄ x 7 H₂O | 5 |
| KCI | 2 |
| Hefeextrakt (Fa. Merck, Darmstadt) | 2 |
| Proteose Pepton (Fa. Difco Laboratories, Detroit, USA) | 7.5 |
| Agar-Agar | 20 |

### Gewinnung des Polymers:

Der gebildete Schleim wurde von der Oberfläche der Petrischalen (handelsübliche Plastik-Petrischalen mit 8,5 cm Durchmesser) geschabt (0,7 ml pro Platte bzw. 0,8 g Feuchtmasse pro Platte) und in 3 ml H₂O pro Petrischale gelöst. Diese Lösung wurde einer Zentrifugation für 1,5 h bei 100 000 x g unterworfen. Der Überstand wurde entnommen und mit einem Volumen 96 % Ethanol versetzt. Gefälltes, sedimentiertes Polymer wurde nach Dekantieren bei -20°C eingefroren und danach lyophilisiert. Das lyophilisierte Polymer wurde erneut in ca. 1,5 ml H₂O aufgenommen und erneut lyophilisiert. Das Polymer wurde auf diese Weise als feines, watteartiges Pulver gewonnen. Die Ausbeute betrug ca. 15 mg Trockenmasse pro bewachsener Petrischale.

### Beispiel 2

### Isolierung des Polymers aus Flüssigmedium

### Kultivierungsbedingungen:

50 ml Flüssigmedium (Zusammensetzung wie Festmedium (s. Tabelle zu Beispiel 1), jedoch wurde kein Agar-Agar zugesetzt und statt 7,5 g Proteose Pepton wurden 8,0 g Casaminosäuren (Fa. Difco Laboratories, Detroit, USA) eingewogen; das Medium wurde nicht erhitzt und direkt nach Lösen der Komponenten verwendet) wurde mit einer Impföse Zellmaterial von Festmedium (s. Tabelle ) beimpft und bei 42°C unter Schütteln (200 Upm) für 48 h inkubiert.

Die Inkubation erfolgte unter nichtsterilen Bedingungen in offenen Gefäßen. Wiederholte Kontrollausstriche zum Ende der Kultivierungszeit auf Standard I-Festmedium (Fa, Merck, Darmstadt) haben keinen Hinweis auf Kontaminationen erbracht. Unter den aufgeführten Bedingungen gewährleistet die hohe Salzkonzentration trotz der Gegenwart großer Mengen an komplexen Nährmedienbestandteilen das alleinige Wachstum des Archaebakteriums.

Für die Gewinnung von Polymer durch Kultivierung in Flüssigmedium wurde das für die Kultivierung auf Festmedium verwendete Nährmedium durch Austausch von Proteose Pepton (s. Tabelle ) durch Casaminosäuren modifiziert.

### Gewinnung des Polymers:

Die bewachsene Kulturflüssigkeit wurde bei 100 000 x g zur Abtrennung der Zellen zentrifugiert. Der Überstand wurde mit einem Volumen 96 % Ethanol versetzt. Gefälltes, sedimentiertes Polymer wurde nach Dekantieren aufgearbeitet, wie in Beispiel 1 angegeben. Die Ausbeute des auf diese Weise gewonnenen Polymers betrug ca. 2 g pro Liter bewachsenes Flüssigmedium.

Das nach Isolierung aus der Kulturbrühe erhaltene Polymer wurde mit Dünnschichtchromatographie, Gelpermeationschromatographie, SDS-Gelelektrophorese und nach Hydrolyse mit HPLC analysiert. Es wurden Polymere der folgenden Formeln ermittelt:

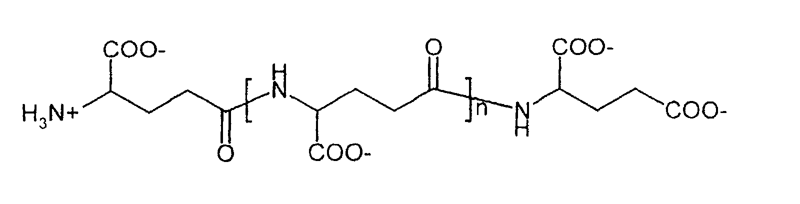

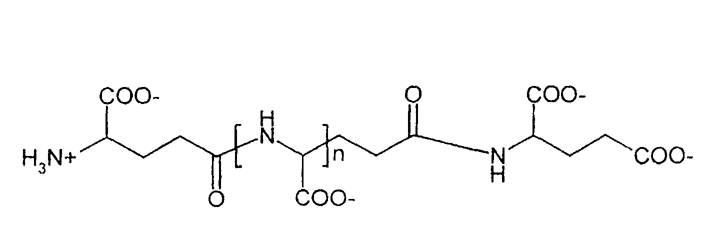

## Patentansprüche

1. Verfahren zur Herstellung von Polyglutaminsäure **dadurch gekennzeichnet, daß** Archaebakterien enthaltende Kulturen eingesetzt werden.

2. Verfahren nach Anspruch 1 **dadurch gekennzeichnet, daß** halophile Archaebakterien enthaltende Kulturen eingesetzt werden.

3. Verfahren nach einem der Ansprüche 1 oder 2 **dadurch gekennzeichnet, daß** das Archaebakterium mit der Hinterlegungsnummer DSMZ 11921 eingesetzt wird.

4. Verfahren nach einem der Ansprüche 1 bis 3 **dadurch gekennzeichnet, daß** synthetische, halbsynthetische oder komplexe Substrate eingesetzt werden.

5. Verfahren nach einem der Ansprüche 1 bis 4 **dadurch gekennzeichnet, daß** anorganische Verbindungen und/oder organische Verbindungen enthaltenden Substrate eingesetzt werden.

6. Verfahren nach einem der Ansprüche 1 bis 5 **dadurch gekennzeichnet, daß** kohlenstoffhaltige sowie stickstoffhaltige Verbindungen, anorganische Salze, Spurenelemente und/oder Vitamine enthaltende Substrate eingesetzt werden.

7. Verfahren nach einem der Ansprüche 1 bis 6 **dadurch gekennzeichnet, daß** Hefeextrakt, Proteose Pepton und Casaminsäuren oder Gemische zweier oder mehrerer dieser Substanzen enthaltende Substrate eingesetzt werden.

8. Verfahren nach einem der Ansprüche 1 bis 7 **dadurch gekennzeichnet, daß** Sulfate, Nitrate, Chloride, Carbonate und Phoshate der Metalle Natrium, Kalium, Magnesium, Mangan, Calcium, Zink und Eisen oder Gemische zweier oder mehrerer dieser Verbindungen enthaltende Substrate eingesetzt werden.

9. Verfahren nach einem der Ansprüche 1 bis 8 **dadurch gekennzeichnet, daß** die Temperaturen im Bereich von 25 bis 50°C liegen.

10. Verfahren nach einem der Ansprüche 1 bis 9 **dadurch gekennzeichnet, daß** die Kultivierung in einem offenen System unter nichtsterilen Bedingungen durchgeführt wird.

11. Archaebakterium mit der Hinterlegungsnummer DSM 11921.

12. Verwendung des Archaebakteriums gemäß Anspruch 11 zur Herstellung von Polyglutaminsäure.

## Claims

1. Process for the production of polyglutamic acid, **characterized in that** cultures comprising archaebacteria are employed.

2. Process according to Claim 1, **characterized in that** cultures comprising halophilic archaebacteria are employed.

3. Process according to one of Claims 1 or 2, **characterized in that** the archaebacterium having the deposit number DSMZ 11921 is employed.

4. Process according to one of Claims 1 to 3, **characterized in that** synthetic, semi-synthetic or complex substrates are employed.

5. Process according to one of Claims 1 to 4, **characterized in that** substrates comprising inorganic compounds and/or organic compounds are employed.

6. Process according to one of Claims 1 to 5, **characterized in that** substrates comprising carbon-containing and nitrogen-containing compounds, inorganic salts, trace elements and/or vitamins are employed.

7. Process according to one of Claims 1 to 6, **characterized in that** substrates comprising yeast extract, proteose peptone and casamino acids or mixtures of two or more of these substances are employed.

8. Process according to one of Claims 1 to 7, **characterized in that** substrates comprising sulphates, nitrates, chlorides, carbonates and phosphates of the metals sodium, potassium, magnesium, manganese, calcium, zinc and iron or mixtures of two or more of these compounds are employed.

9. Process according to one of Claims 1 to 8, **characterized in that** the temperatures are in the range from 25 to 50°C.

10. Process according to one of Claims 1 to 9, **characterized in that** the culturing is carried out in an open system under non-sterile conditions.

11. Archaebacterium having the deposit number DSM 11921.

12. Use of the archaebacterium according to Claim 11, for the production of polyglutamic acid.

## Revendications

1. Procédé de préparation de poly(acide glutamique), **caractérisé en ce que** l'on met en oeuvre des cultures contenant des Archaebactéries.

2. Procédé suivant la revendication 1, **caractérisé en ce que** l'on met en oeuvre des cultures contenant des Archaebactéries halophiles.

3. Procédé suivant la revendication 1 ou 2, **caractérisé en ce que** l'on met en oeuvre des Archaebactéries avec le numéro de dépôt DSMZ 11921.

4. Procédé suivant l'une quelconque des revendications 1 à 3, **caractérisé en ce que** l'on met en oeuvre un substrat synthétique, semi-synthétique ou complexe.

5. Procédé suivant l'une quelconque des revendications 1 à 4, **caractérisé en ce que** l'on met en oeuvre des substrats contenant des composés inorganiques et/ou des composés organiques.

6. Procédé suivant l'une quelconque des revendications 1 à 5, **caractérisé en ce que** l'on met en oeuvre des substrats contenant des composés carbonés ainsi que azotés, des sels inorganiques, des éléments trace et/ou des vitamines.

7. Procédé suivant l'une quelconque des revendications 1 à 6, **caractérisé en ce que** l'on met en oeuvre des substrats contenant de l'extrait de levure, la protéose de peptone et des acides casaminés ou des mélanges de deux ou plusieurs de ces substances.

8. Procédé suivant l'une quelconque des revendications 1 à 7, **caractérisé en ce que** l'on met en oeuvre des substrats contenant des sulfates, nitrates, chlorures, carbonates et phosphates des métaux sodium, potassium, magnésium, manganèse, calcium, zinc et fer ou des mélanges de deux ou plusieurs de ces composés.

9. Procédé suivant l'une quelconque des revendications 1 à 8, **caractérisé en ce que** la température se situe dans l'intervalle allant de 25 à 50°C.

10. Procédé suivant l'une quelconque des revendications 1 à 9, **caractérisé en ce que** l'on réalise la culture dans un système ouvert dans des conditions non stériles.

11. Archaebactéries avec le numéro de dépôt DSM 11921.

12. Utilisation des Archaebactéries suivant la revendication 11, pour préparer du poly(acide glutamique).
